# EUROPÄISCHE PATENTSCHRIFT

(11) **EP 1 227 991 B1**
(45) Veröffentlichungstag und Bekanntmachung des Hinweises auf die Patenterteilung: **15.12.2010**
(21) Anmeldenummer: 00978974.4
(22) Anmeldetag: 24.10.2000
(51) Int. Cl.: B65D 65/02, A61F 13/15

(54) **VERFAHREN ZUM VERPACKEN VON GEGENSTÄDEN UND BAHNFÖRMIGES VERPACKUNGSMATERIAL**
METHOD FOR PACKAGING OBJECTS AND A PACKAGING MATERIAL IN THE FORM OF A STRIP
PROCEDE D'EMBALLAGE D'OBJETS ET MATERIAU D'EMBALLAGE EN FORME DE BANDE

(30) Priorität: 02.11.1999 DE 19952569
(43) Veröffentlichungstag der Anmeldung: 07.08.2002
(73) Patentinhaber: Huhtamaki Forchheim Zweigniederlassung der Huhtamaki Deutschland GmbH & Co. KG, 91301 Forchheim (DE)
(72) Erfinder: SCHMIDT, Werner, 91301 Forchheim (DE); KREUDER, Reinhard, 91301 Forchheim (DE)
(74) Vertreter: Epping - Hermann - Fischer
(86) Internationale Anmeldenummer: PCT/EP2000/010473
(87) Internationale Veröffentlichungsnummer: WO 2001/032526

(56) Entgegenhaltungen:
- WO-A-98/53781
- WO-A-99/60965
- DE-A- 3 541 753
- GB-A- 2 298 627
- US-A- 5 333 753

## Beschreibung

Die Erfindung bezieht auf ein Verfahren zum Verpacken von wenigstens partiell selbstklebend ausgerüsteten Gegenständen, mittels eines Verpackungsmaterials, welches abschnittsweise mit einer Antihaft-Ausrüstung versehen ist, wobei die Gegenstände mit ihrem selbstklebenden Abschnitt auf die Abschnitte mit Antihaft-Ausrüstung des Verpackungsmaterials aufgelegt werden und die Gegenstände anschließend durch Verpackungsmaterial abgedeckt werden.

Aus GB-A-2 298 627 ist ein Verfahren zum Verpacken von Gegenständen ähnlicher Art bekannt.

Es sind einige Gegenstände denkbar, die selbstklebend ausgerüstet sind und deren Klebeflächen während der Lagerung geschützt werden müssen, um ihre Klebkraft zu erhalten. Hierzu werden diese meist mit separaten Abdeckstreifen belegt und dann der komplette Gegenstand verpackt.

Der Erfindung liegt die Aufgabe zugrunde, ein Verfahren aufzuzeigen, mit dem in einfacher Weise sowohl die selbstklebende Ausrüstung geschützt als auch der ganze Gegenstand verpackt und auf einfache Art und Weise aus der Verpackung entnommen werden kann.

Diese Aufgabe wird erfindungsgemäß dadurch gelöst, daß zum Abdecken der Gegenstände eine weitere Verpackungsmaterial-Bahn eingesetzt wird und daß am Rand der Verpackung wenigstens eine freie Lasche vorgesehen wird, die von der Verpackungsmaterial-Bahn abgetrennt und unter den einen Endabschnitt der zu verpackenden Gegenstände gelegt wird.

Dadurch ist das Einpacken des Gegenstandes ebenfalls besonders einfach durchzuführen, wobei unterschiedliche oder auch gleiche Verpackungsmaterialien für beide Verpackungsseiten verwendet werden können. Zudem kann die Verpackung leicht geöffnet und der verpackte Gegenstand auch wieder leicht aus der Verpackung entnommen werden.

Als ebenfalls sehr vorteilhaft hat es sich erwiesen, wenn erfindungsgemäß nach dem Ein- bzw. Auflegen der Gegenstände Einzelverpackungen abgeteilt werden.

Trotz der Abteilung können die Einzelverpackungen zusammenhängend verbleiben und können erst später abgetrennt werden.

Die Abteilung der Einzelverpackungen wird gemäß einer weiteren vorteilhaften Ausgestaltung der Erfindung durch eine sogenannte Verkrümperung durchgeführt.

Gemäß einer weiteren vorteilhaften Ausgestaltung der Erfindung werden zum Erleichtern des Abtrennens von Einzelpackungen Perforationslinien vorgesehen.

Damit ist ein besonders einfaches Abtrennen der Einzelpackungen ermöglicht.

Eine weitere vorteilhafte Ausgestaltung des erfindungsgemäßen Verfahrens liegt darin, daß mit zwei klebenden Abschnitten versehene Wund-Pflaster verpackt werden, wobei wenigstens im Bereich der klebenden Abschnitte die Abschnitte mit Antihaft-Ausrüstung vorgesehen sind.

Eine weitere vorteilhafte Ausgestaltung des erfindungsgemäßen Verfahrens ist dadurch gekennzeichnet, daß Slip-Einlagen verpackt werden, die mit wenigstens einem selbstklebenden Abschnitt versehen sind, in dessen Bereich ein Abschnitt mit Antihaft-Ausrüstung vorgesehen ist.

Weiterhin sehr vorteilhaft ist eine Ausgestaltung der Erfindung, gemäß der Damenbinden verpackt werden, die mit wenigstens einem selbstklebenden Abschnitt versehen sind, in dessen Bereich ein Abschnitt mit Antihaft-Ausrüstung vorgesehen ist.

Eine weitere vorteilhafte Ausgestaltung des erfindungsgemäßen Verfahrens besteht darin, daß die Außenseite der Verpackungshülle eine textilähnliche Aufinachung erhält, und daß die Antihaft-Ausrüstung auf der Innenseite der Verpackungshülle abschnittsweise vorgesehen wird.

Sehr vorteilhaft ist es dabei, wenn gemäß einer weiteren Ausgestaltung der Erfindung die Verpackungshülle von einer Kunststoffolie gebildet wird, deren Außenseite eine Prägung erhält, die ihr ein textilähnliches Aussehen verleiht.

Vorteilhaft ist es dabei auch, wenn die Verpackungshülle von einer äußeren geprägten und einer inneren glatten Kunststofffolie gebildet wird.

Eine weitere vorteilhafte Ausgestaltung der Erfindung liegt darin, daß die Verpackungshülle von einem Vliesstoff enthält.

Als sehr vorteilhaft hat es sich auch erwiesen, wenn gemäß einer weiteren Ausgestaltung der Erfindung der Vliesstoff vorzugsweise an seiner Innenseite mit einer Kunststofffolie verbunden wird.

Durch alle diese Ausgestaltungen wird eine komfortable Außenseite der Verpackungshülle geschaffen, die entsprechend den jeweiligen Ansprüchen an Dichtigkeit ausgestaltet werden kann.

Eine weitere vorteilhafte Ausgestaltung der Erfindung liegt darin, daß wenigstens ein Randstreifen der Verpackungsmaterialbahn von Antihaft-Material frei gehalten wird.

Damit ist ein einfaches Verschließen der Verpackung an ihren Rändern gewährleistet.

Sehr vorteilhaft ist auch eine weitere Ausgestaltung der Erfindung, gemäß der die Gegenstände auf einen in der Mitte der Verpackungsmaterialbahn angeordneten durchgehenden oder unterbrochenen Längsstreifen mit Antihaft-Ausrüstung aufgelegt werden.

Als sehr vorteilhaft hat es sich auch ergeben, wenn gemäß einer weiteren Ausgestaltung der Erfindung die Gegenstände auf ein Muster aus abwechselnd mehreren parallelen Längsstreifen mit Antihaft-Ausrüstung und Längsstreifen, die frei von Antihaft-Ausrüstung sind, aufgelegt werden.

Ebenfalls sehr vorteilhaft ist es, wenn gemäß einer weiteren Ausgestaltung der Erfindung die Gegenstände auf einzelne Abschnitte mit Antihaft-Ausrüstung , die entsprechend den zu verpackenden Gegenständen bzw. deren Kleberbeschichtung angeordnet sind, aufgelegt werden.

Als sehr günstig hat es sich auch erwiesen, wenn gemäß einer weiteren Ausgestaltung der Erfindung zur Bildung der Antihaft-Ausrüstung Polysiloxane eingesetzt werden, die gehärtet werden.

Eine weitere vorteilhafte Ausgestaltung der Erfindung ist dadurch gekennzeichnet, daß als Verpackungsmaterialbahn eine Kunststoffolie eingesetzt wird, die als Mono- oder Mehrschichtfolie ausgebildet ist und geprägt sein kann.

Gemäß einer weiteren Ausgestaltung der Erfindung kann als Verpackungsmaterialbahn jedoch auch ein vorzugsweise wenigstens einseitig mit Kunststoff beschichtetes Papier eingesetzt werden.

Sehr vorteilhaft ist es auch, wenn gemäß einer weiteren Ausgestaltung der Erfindung auf die von der Antihaft-Ausrüstung abgekehrte Seite der Verpackungsmaterialbahn ein Druckbild aufgebracht wird.

In der Zeichnung ist die Erfindung anhand mehrerer Ausführungsbeispiele veranschaulicht. Dabei zeigen:
- Fig. 1: eine Verpackungsmaterialbahn im flachliegenden Zustand, mit querverlaufenden, silikonisierten Abschnitten und auf diese aufgelegten Wundpflastern,
- Fig.2: die Verpackungsmaterialbahn nach Fig.1 im zusammengefalteten Zustand,
- Fig.3: eine weitere Verpackungsmaterialbahn mit abschnittsweise vorgesehenen silikonisierten Flächen, auf welche Wundpflaster aufgelegt sind,
- Fig.4: die Verpackungsmaterialbahn nach Fig.3 um ihre Längsmittelachse zusammengelegt,
- Fig.5: eine Verpackungsmaterialbahn gemäß der Erfindung mit quer verlaufenden silikonisierten Abschnittenund darauf liegenden Pflastern,
- Fig.6: eine aus der Materialbahn nach Fig.5 entlang von Längsfaltlinien gefalteten Verpackungsbahn,
- Fig.7: eine Materialbahn mit Teilsilikonisierungen und abgetrenntem Randstreifen, der unter die eingelegten Pflaster gelegt ist,
- Fig.8: ein aus der Verpackungsmaterialbahn nach Fig. 7 Verpackungsstreifen,
- Fig.9: eine Verpackungsmaterialbahn mit streifenförmigen Silikonabschnitten, darauf aufgelegten Pflastern sowie einer weiteren Verpackungsmaterialbahn zum Abdecken der Pflaster,
- Fig.10: eine Verpackungsmaterialbahn mit in Längsrichtung verlaufenden silikonisierten Abschnitten und darauf aufgelegten Pflastern,
- Fig.11: eine Verpackungsbahn, hergestellt durch Falten der Materialbahn nach Fig.10,
- Fig. 12: einen Vertikalschnitt durch einen Materialabschnitt, mit einer Kunststoffolie, die einseitig mit einem Vlies und auf der anderen Seite mit einer partiell aufgetragenen Antihaftbeschichtung versehen ist,
- Fig.13: einen Vertikalschnitt durch einen weiteren Materialabschnitt, der einschichtig aus einem Vlies gebildet ist, das auf der späteren Innenseite der Verpackungshülle mit einer partiellen Antihaftbeschichtung versehen ist,
- Fig. 14: einen Vertikalschnitt durch einen Materialabschnitt, der aus einer geprägten Kunststoffolie besteht,
- Fig.15: einen Abschnitt einer Verpackungsmaterialbahn mit einer Antihaft²- Beschichtung und einem Randstreifen, der frei von dieser Beschichtung und siegelbar ist,
- Fig.16: einen Abschnitt einer weiteren Verpackungsmaterialbahn mit einem mittleren Längsstreifen mit Antihaftbeschichtung und zwei Randstreifen, die frei von Antihaftbeschichtung sind,
- Fig.17: einen Abschnitt einer weiteren Verpackungsmaterialbahn mit drei Längsstreifen mit Antihaftbeschichtung, die jeweils von Streifen ohne Antihaftbeschichtung längsseits eingerahmt sind,
- Fig.18: einen Abschnitt einer weiteren Verpackungsmaterialbahn mit in Längsrichtung voneinander beabstandeten Antihaftbeschichtungs- Abschnitten und
- Fig.19: einen Abschnitt einer Verpackungsmaterialbahn mit einzelnen Abschnitten einer Antihaftbeschichtung.

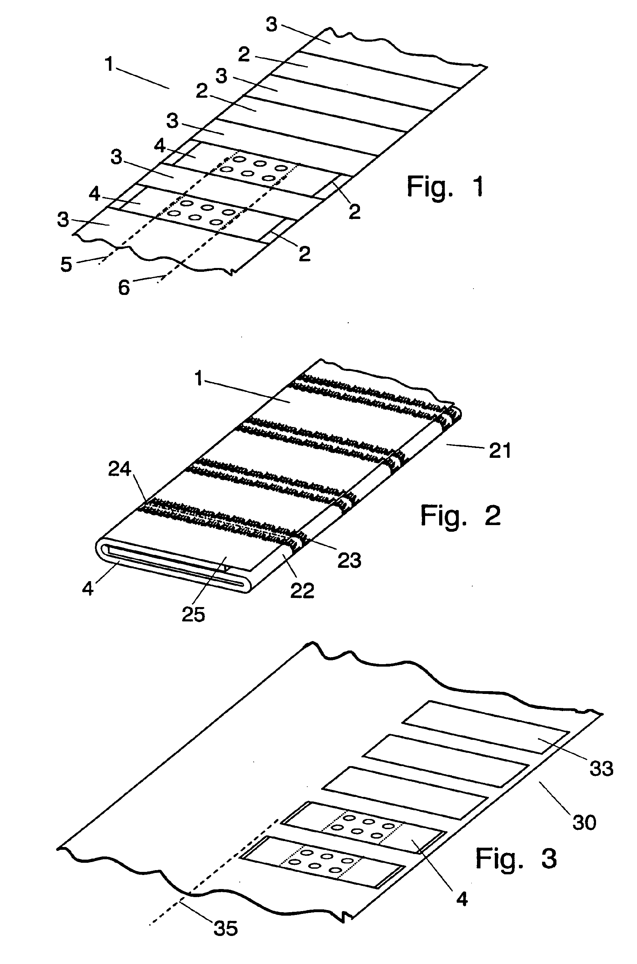

In Fig.1 ist eine Verpackungsmaterialbahn 1 dargestellt, die mit quer zur Bahnlängsrichtung verlaufenden und sich über die ganze Bahnbreite erstreckenden Streifen 2 mit Silikonbeschichtung versehen ist, zwischen denen Streifen 3 ohne Silikonbeschichtung verbleiben.

Auf die mit Silikon beschichteten Streifen 2 sind Wundpflaster 4 aufgelegt, die mit selbstklebenden Abschnitten versehen sind, welche auf dem Silikon nur schwach haften und damit wieder leicht ablösbar sind. Mit 5 und 6 sind zwei Faltlinien bezeichnet, um welche die beiden Seitenabschnitte der Verpackungsmaterialbahn 1 auf deren Mittelabschnitt gefaltet werden.

Dabei ergibt sich dann ein Verpackungsstreifen 21, wie er in Fig.2 in größerem Maßstab dargestellt ist. Dieser Verpackungsstreifen 21 ist unmittelbar neben einem Wundpflaster 4 abgeschnitten um dessen Einlagerung erkennbar zu machen. Im freien Zwischenraum 3 zwischen zwei Wundpflastern 4 sind zwei quer verlaufende Verkrümperungsstreifen 22 und 23 angeordnet, mit denen Einzelverpackungen für jeweils ein Pflaster 4 abgeteilt sind. Zwischen diesen beiden Verkrümperungsstreifen 22,23 ist noch eine Perforationslinie 24 vorgesehen, entlang welcher Einzelverpackungen abtrennbar sind.

Anstelle der Perforationslinien 24 können Einzelverpackungen auch einfach abgeschnitten werden.

Es ist auch möglich nur einen einzigen Verkrümperungsstreifen vorzusehen innerhalb dem die Einzelverpackungen dann abgetrennt werden.

Ein nicht vom Wundpflaster 4 abgedeckter Randstreifen 25 der Verpackungsmaterialbahn 1 ist frei zugänglich und kann zum Öffnen der Einzelverpackung leicht ergriffen werden. Dieser Randstreifen 25 kann auch angeklebt sein, sollte aber leicht abzulösen sein.

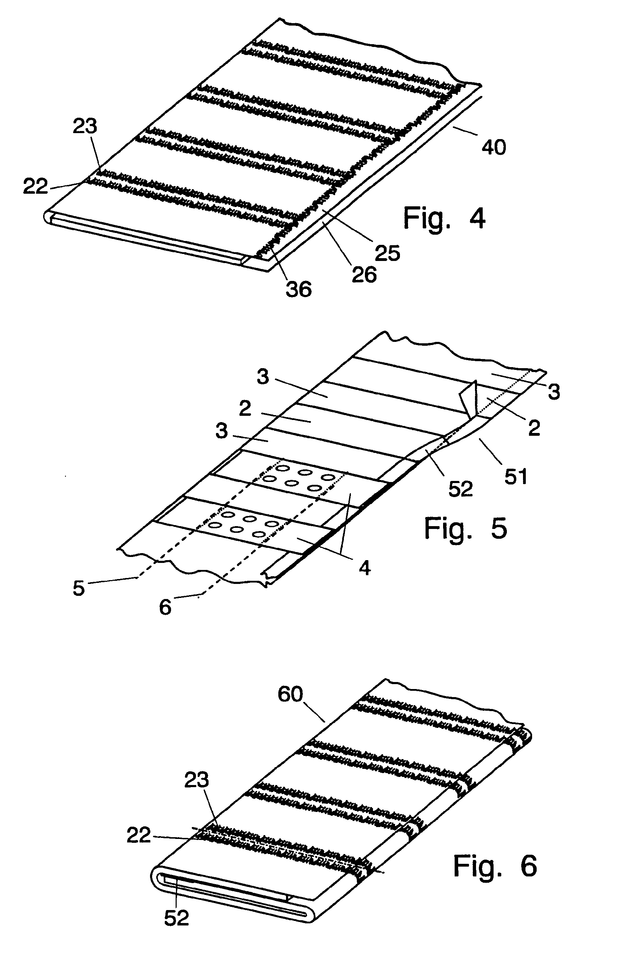

Beim Ausführungsbeispiel nach Fig.3 ist eine Verpackungsmaterialbahn 30 vorgesehen, die nur in der einen Hälfte mit silikonisierten Streifen 33 versehen ist, auf welche Wundpflaster 4 mit ihren selbstklebenden Abschnitten aufgeklebt sind. Entlang einer Mittel-Faltlinie 35 wird die Verpackungsmaterialbahn 30 zu einer Verpackung 40 zusammengefaltet, wie dies in Fig.4 gezeigt ist. Dabei liegt das Wundpflaster 4 auf der einen Seite der Verpackung 40 auf und ist durch die andere Seite abgedeckt.

Auch bei dieser Ausgestaltung sind wieder zwei Verkrümperungsstreifen 22 und 23 vorgesehen, die zum Verschließen von Einzelverpackungen dienen und zwischen denen diese Einzelverpackungen abgetrennt werden können. Eine weitere längs verlaufende Verkrümperungsnaht 36 dient dazu, die Verpackungen allseitig zu verschließen. Diese Verkrümperungsnaht 36 ist mit Abstand von der Außenkante der Verpackung 40 angeordnet, wodurch zwei freiliegende Laschen 25 und 26 verbleiben, welche zum Öffnen der Einzelverpackungen leicht zu Ergreifen sind. Anstelle der Verkrümperungsnaht 36 kann auch eine Klebenaht vorgesehen werden.

Das Ausführungsbeispiel nach den Fig.5 und 6 entspricht weitgehend dem Beispiel gemäß Fig.1 und 2; lediglich wird vor dem Auflegen der Wundpflaster 4 auf die silikonisierten Streifen 2 von der Verpackungsmaterialbahn 51 ein Randstreifen 52 abgetrennt und unter den einen Endabschnitt der Wundpflaster gelegt. Damit lassen sich die Wundpflaster 4 besonders einfach von der Verpackungsmaterialbahn abziehen.

In Fig.6 ist eine Verpackung 60 dargestellt, die aus der Verpackungsmaterialbahn 51 aufgefaltet ist. Auch hier sind Verkrümperungsstreifen 22 und 23 angeordnet, welche Einzelverpackungen abteilen und in deren Bereich die Einzelverpackungen abgetrennt werden können.

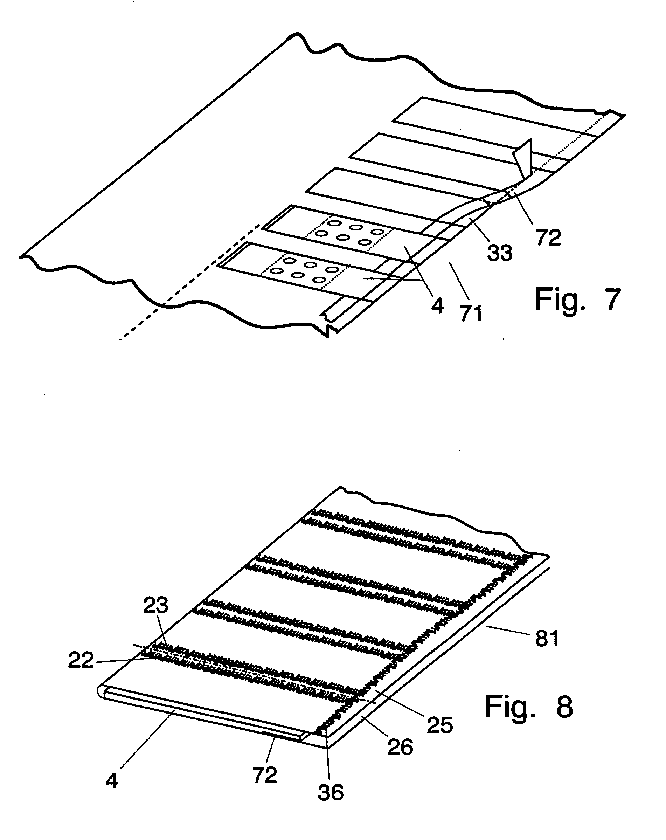

Beim Ausführungsbeispiel nach Fig.7 reichen die silikonisierten Streifen 33 wieder nur über die halbe Breite der Verpackungsmaterialbahn 71, gehen aber bis zum äußeren Rand durch.

Vor dem Auflegen der Wundpflaster 4 wird auch hier ein Randstreifen 72 von der Verpackungsmaterialbahn 71 abgeschnitten und so auf die Materialbahn aufgelegt, daß ein klebender Abschnitt des Wundpflasters auf dem Randstreifen aufliegt.

Die Verpackungsmaterialbahn 71 wird dann um ihre Mittellängsachse gefaltet, wobei die Wundpflaster völlig abgedeckt werden.

Zum Abteilen und Verschließen der Einzelverpackungen sind wieder quer und längs verlaufende Verkrümperungsstreifen 22,23 und 36 vorgesehen, wie dies in Fig.8 anhand der fertigen Verpackung 81 dargestellt ist.

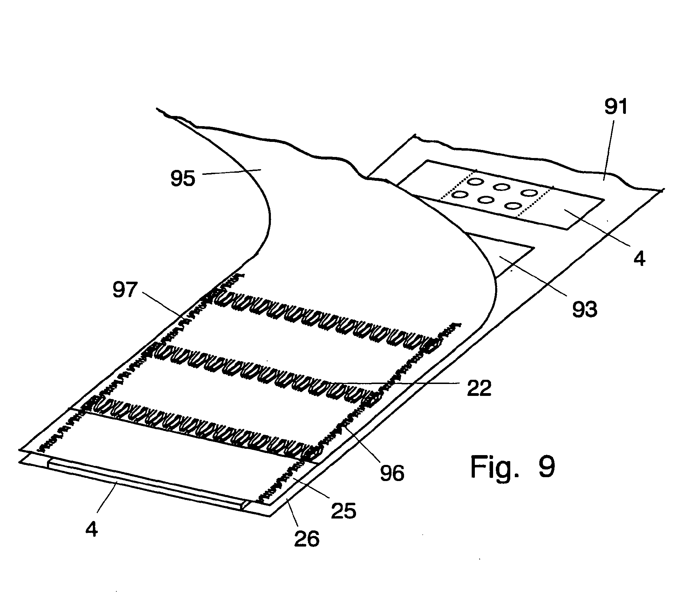

In Fig.9 ist ein Ausführungsbeispiel dargestellt, mit einer Verpackungsmaterialbahn 91, die mit quer verlaufenden, silikonisierten Streifen 93 versehen sind. Auf diese silikonisierten Streifen 93 werden die Wundpflaster 4 aufgelegt. Dann wird eine weitere Verpackungsmaterialbahn 95 aufgelegt und beide Bahnen 91 und 95 mittels zweier längsverlaufender Verkrümperungsstreifen 96 und 97 mit einander verbunden. Durch einen quer verlaufenden Verkrümperungsstreifens 22 sind Einzelverpackungen abgeteilt, die durch Abreissen entlang einer nicht dargestellten Perforationslinie oder durch Abschneiden abgetrennt werden können. Die beiden außerhalb des einen längsverlaufenden Verkrümperungsstreifens liegenden freien Laschen 25 und 26 dienen zum leichteren Öffnen der Einzelverpackung.

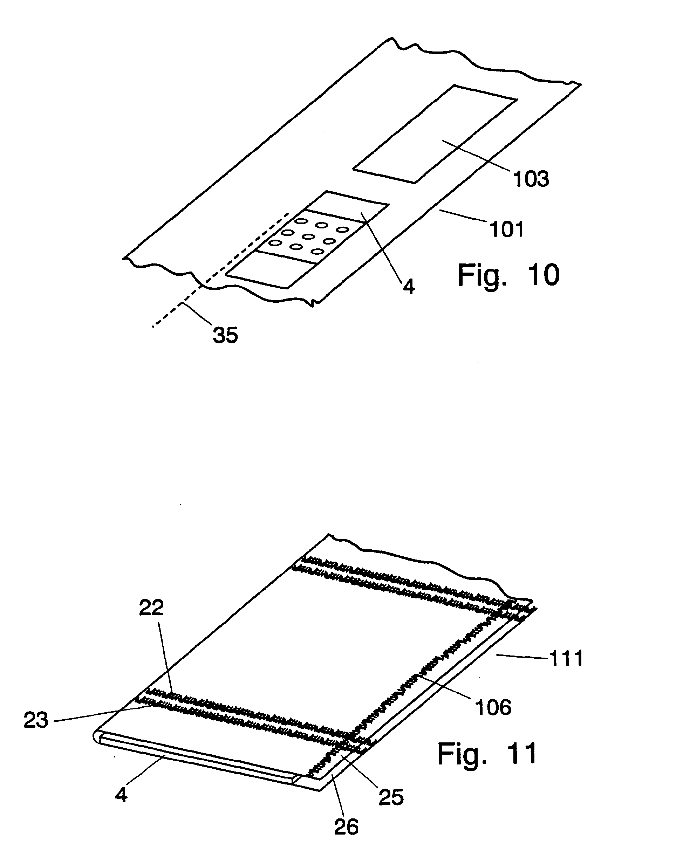

Die Fig.10 zeigt ein Ausführungsbeispiel einer Verpackungsmaterialbahn 101, die mit in Längsrichtung der Bahn verlaufenden silikonisierten Streifen 103 versehen ist. Auf diese Streifen 103 sind dann ebenfalls in Längsrichtung ausgerichtet Wundpflaster 4 aufgelegt. Um die Längsmittelachse 35 wird dann die Verpackungsmaterialbahn 101 zusammengefaltet, wie dies in Fig.11 dargestellt ist. Die dabei entstehende Verpackung 111 ist durch quer verlaufende Verkrümperungsstreifen 22 und 23 zu Einzelverpackungen abgeteilt, die zusätzlich durch eine längs verlaufende Verkrümperungsnaht 106 verschlossen sind. Dabei verbleiben außerhalb dieser Verkrümperungsnaht 106 die beiden Griff-Laschen 25 und 26 zum Öffnen der Einzelverpackungen.

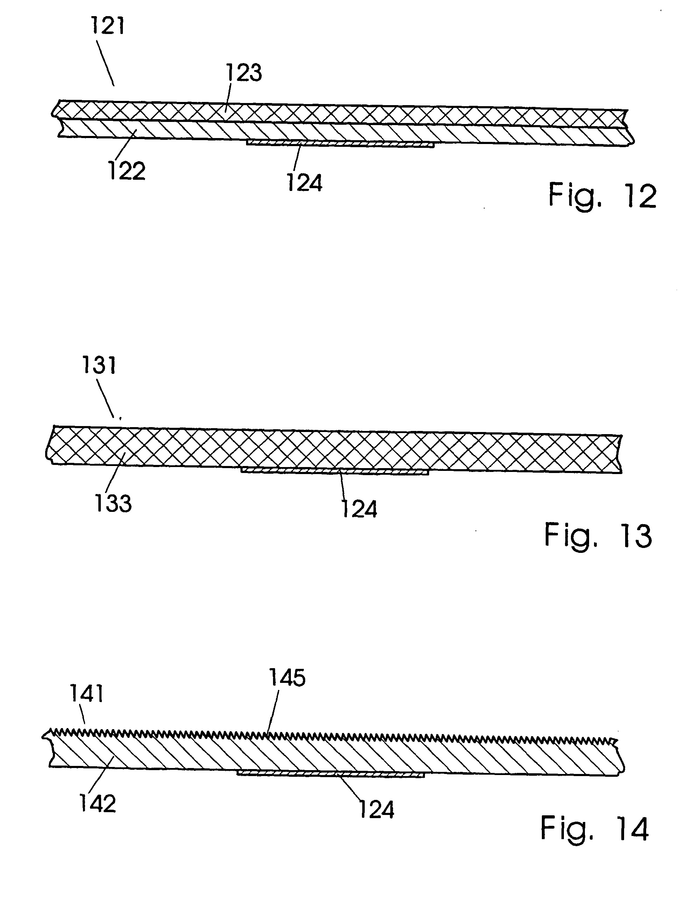

Mit 121 ist in Fig.12 ein Materialabschnitt bezeichnet, der zum Herstellen einer Verpackungshülle vorgesehen ist, die ihrerseits zum Verpacken von Damenbinden dienen soll. Der Materialabschnitt 121 besteht aus einer Kunststoffolie 122, auf deren einer Seite ein Vlies 123 aufgebracht ist. Auf der vom Vlies abgewandten Seite ist die Kunststoffolie mit einer Anthaftbeschichtung 124 versehen, die nur partiell aufgebracht ist. Beim Zusammenfalten des Materialabschnittes 121 kommt die Antihaftbeschichtung 124 nach innen und ermöglicht ein nur leichtes Ankleben der mit einer klebenden Seite ausgerüsteten Damenbinde. Durch die Faltung des Materialabschnittes 121 ergeben sich auf der Innenseite der Verpackung Bereiche, in denen wenigstens zwei Lagen der Kunststoffolie 122 direkt aufeinander zu liegen kommen. In diesen Bereichen werden die Lagen der Kunststoffolie 122 miteinander versiegelt oder verkrümpert. Das Versiegeln oder Verkrümpern wird durch die Antihaftbeschichtung 124 nicht beeinträchtigt. Die nur leicht an der Antihaftbeschichtung 124 anhaftende Damenbinde läßt sich leicht aus der Verpackung entnehmen.

Der in Fig.13 dargestellte Materialabschnitt 131 besteht aus einem Vlies 133. Dieses Vlies 133 ist auf seiner einen Seite partiell mit einer Antihaftbeschichtung 124 versehen. Durch Zusammenfalten des Materialabschnittes kommt die Antihaftbeschichtung 124 ebenso wie beim oben angeführten Ausführungsbeispiel nach innen. Die klebende Seite einer Damenbinde haftet nur leicht an der Antihaftbeschichtung 124 an. Eine leichte Entnehmbarkeit der Damenbinde aus der Verpackung ist somit gewährleistet. Ebenso ist durch die nur partielle Antihaftbeschichtung 124 die Siegel- und Krümperfähigkeit der zusammengefalteten Vlies-Lagen sichergestellt.

Das in Fig.14 dargestellte weitere Ausführungsbeispiel zeigt einen Materialabschnitt 141. Dieser Materialabschnitt 141 besteht aus einer Kunststoffolie 142, auf deren einer Seite eine textilähnliche Prägung 145 angeordnet ist. Auf der der Prägung 145 abgewandten Seite ist die Folie 142 mit einer partiell aufgebrachten Antihaftbeschichtung 124 versehen. Die Antihaftbeschichtung 124 kommt beim Zusammenfalten des Materialabschnittes 141 nach innen. Durch die nur partiell aufgebrachte Antihaftbeschichtung 124 ist in den von der Antihaftbeschichtung 124 ausgesparten Bereichen eine Aneinandersiegelung oder Verkrümperung der sich beim Zusammenfalten bildenden mehreren Lagen des Materialabschnittes 141 ohne Probleme möglich. Die durch die Verpackung umschlossenen Damenbinde läßt sich aufgrund des nur leichten Anhaftens ihrer Klebeseite an der Antihaftbeschichtung 124 leicht aus der Verpackung herausnehmen.

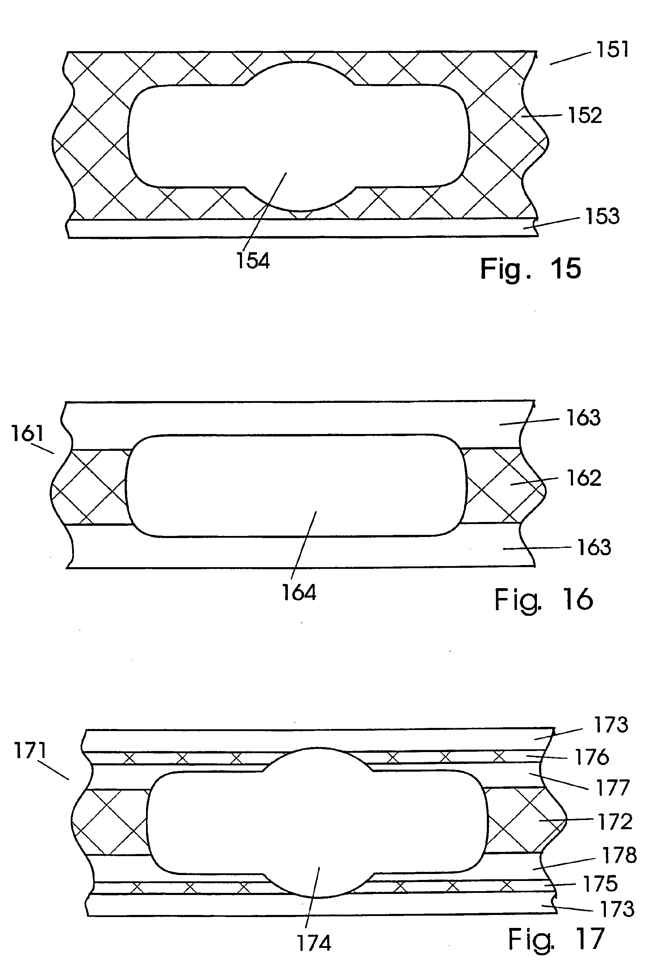

Mit 151 ist in Fig.15 eine weitere Verpackungsmaterialbahn bezeichnet, die mit einer Antihaftschicht 152 sowie einem Randstreifen 153 versehen ist, wobei der Randstreifen 153 frei von einer Antihaftbeschichtung ist. Auf die Antihaftbeschichtung 152 wird - wie in Fig.15 dargestellt - ein Gegenstand 154 aufgelegt, der in einen entsprechenden Abschnitt der Verpackungsmaterialbahn eingeschlagen werden soll. Der Gegenstand 154 - beim dargestellten Ausführungsbeispiel eine Damenbinde mit seitlichen Flügeln - ist auf seiner Unterseite mit einem Haftkleber versehen, der zwar auf der Antihaftbeschichtung 152 klebt aber sich leicht wieder ablösen läßt ohne seine Klebkraft zu verlieren. Zwei seitliche Abschnitte der Verpackungsmaterialbahn werden zum Verpacken der Damenbinde über diese gefaltet und die übereinanderliegenden Randstreifen 153 miteinander versiegelt. Der gegenüberliegende Rand wird dann eingefaltet und die Verpackung in nicht dargestellter Weise geschlossen.

Anstelle einer Versiegelung der Randstreifen ist auch ein Kleben oder eine Verkrümperung möglich.

Beim Ausführungsbeispiel nach Fig.16 weist die Verpackungsmaterialbahn 161 einen mittleren Längsstreifen 162 mit Antihaftbeschichtung auf, der seitlich von zwei Randstreifen 163 eingerahmt ist. Auf diesen Antihaft-Längsstreifen 162 kann wieder eine Damenbinde ohne selbstklebende Flügel aufgelegt werden. Bei entsprechender Ausgestaltung kann diese Verpackungsmaterialbahn 161 auch zum Verpacken von Wundpflaster 164 eingesetzt werden.

Zum Verschließen einer Verpackung aus einem Abschnitt dieser Verpackungsmaterialbahn 161 werden wieder die seitlichen Abschnitte mit einer gegenseitigen Überlappung eingefaltet. Durch Verbinden der beiden Randstreifen 163 wird dann eine weitgehend dichte Verpackung erzielt.

Die Verpackungsmaterialbahn 171 gemäß Fig.17 dient wieder dem Verpacken einer Damenbinde 174 mit Flügeln, bei der die Flügel mit Kleberstellen versehen sind. Die Verpackungsmaterialbahn 171 ist mit drei Längsstreifen 172, 175 und 176 mit Releasebeschichtung versehen, die durch zwei Längsstreifen 177 und 178 voneinander getrennt sind. Diese Längsstreifen 177 und 178 sind genauso wie die beiden Randstreifen 173 frei von einer Antihaftbeschichtung, so daß zumindest die Randstreifen miteinander verbunden werden können.

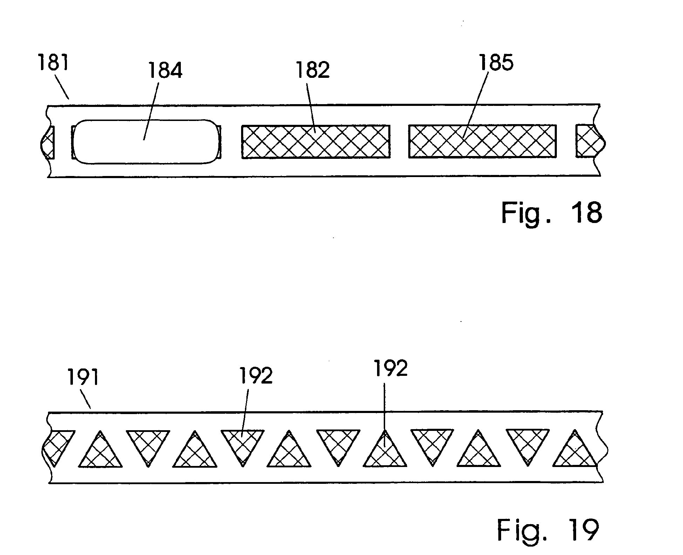

In Fig.18 ist ein Ausführungsbeispiel einer Verpackungsmaterialbahn 181 dargestellt, bei der die Antihaftbeschichtungen 182, 185 in Längsrichtung unterbrochen und nur in dem Bereich angeordnet sind, in welchem sie für den Haftkleber des jeweiligen Gegenstandes benötigt werden. Die einzelnen Abschnitte der Antihaftbeschichtung sind dabei in Längsrichtung hintereinander angeordnet. Neben der Randstreifen-Verbindung ist hierbei auch eine Verbindung innerhalb der releasefreien Querabschnitte möglich, so daß eine allseits dichte Verpackung geschafffen werden kann.

Die Fig.19 zeigt dagegen ein Ausführungsbeispiel einer Verpackungsmaterialbahn 191, bei der die Abschnitte der Antihaftbeschichtung 192 lediglich entsprechend den durch den zu verpackenden Gegenstand gegebenen Notwendigkeiten angeordnet sind.

Als Material für die Verpackungsmaterialbahn kommen Kunststoffolien in Frage, die als Monofolien oder als Mehrschichtfolien ausgebildet sind. Darüber hinaus ist es möglich, Papier zu verwenden. Auch Vliesstoffe können für die Verpackungsmaterialbahn verwendet werden. Sowohl die Papierbahn als auch die Vliesbahn können wenigstens einseitig mit Kunststoff beschichtet werden.

Als Antihaftbeschichtung haben sich Polysiloxane besonders bewährt, wobei sowohl lösemittelhaltige als auch lösemittelfreie Polysiloxane eingesetzt werden können. Die Härtung der Polysiloxane erfolgt entweder thermisch oder durch Strahlenhärtung.

Auf der von der Releaseschicht abgewandten Seite kann die Verpackungsmaterialbahn noch mit einem Druckbild versehen sein.

## Patentansprüche

1. Verfahren zum Verpacken von wenigstens partiell selbstklebend ausgerüsteten Gegenständen(4,154,164,174,184), mittels eines Verpackungsmaterials(91), welches abschnittsweise mit einer Antihaft-Ausrüstung(93) versehen ist, wobei die Gegenstände(4,154,164,174,184) mit ihrem selbstklebenden Abschnitt auf die Abschnitte mit Antihaft-Ausrüstung des Verpackungsmaterials aufgelegt werden und die Gegenstände anschließend durch Verpackungsmaterial abgedeckt werden, **dadurch gekennzeichnet, daß** zum Abdecken der Gegenstände(4) eine weitere Verpackungsmaterial-Bahn(95) eingesetzt wird und daß am Rand der Verpackung wenigstens eine freie Lasche(52) vorgesehen wird, die von der Verpackungsmaterial-Bahn(91) abgetrennt und unter den einen Endabschnitt der zu verpackenden Gegenstände gelegt wird.

2. Verfahren nach Anspruch 1, **dadurch gekennzeichnet, daß** nach dem Ein- bzw. Auflegen der Gegenstände(4,154,164,174,184) Einzelverpackungen abgeteilt werden.

3. Verfahren nach Anspruch 2, **dadurch gekennzeichnet, daß** das Abteilen der Einzelpackungen durch eine sogenannte Verkrümperung ((22,23) durchgeführt wird.

4. Verfahren nach einem der vorhergehenden Ansprüche, **dadurch gekennzeichnet, daß** zum Erleichtern des Abtrennens von Einzelpackungen Perforationslinien vorgesehen werden.

5. Verfahren nach einem der vorhergehenden Ansprüche, **dadurch gekennzeichnet, daß** mit zwei klebenden Abschnitten versehene Wund-Pflaster(4) verpackt werden, wobei wenigstens im Bereich der klebenden Abschnitte die Abschnitte mit Antihaft-Ausrüstung(2,103) vorgesehen sind.

6. Verfahren nach einem der Ansprüche 1 bis 4, **dadurch gekennzeichnet, daß** Slip-Einlagen(154,164,174,184) verpackt werden, die mit wenigstens einem selbstklebenden Abschnitt versehen sind, in dessen Bereich ein Abschnitt mit Antihaft-Ausrüstung(124,152,162,172,175,176,182,185,192) vorgesehen ist.

7. Verfahren nach einem der Ansprüche 1 bis 4, **dadurch gekennzeichnet, daß** Damenbinden verpackt werden, die mit wenigstens einem selbstklebenden Abschnitt versehen sind, in dessen Bereich ein Abschnitt mit Antihaft-Ausrüstung(124,152,162,172,175,176,182,185,192) vorgesehen ist.

8. Verfahren nach einem der vorhergehenden Ansprüche, mit einer Antihaftbeschichtung(124,152,162,172,175,176,182,185,192) auf der Innenseite der durch das Verpackungsmaterial gebildeten Verpackungshülle, **dadurch gekennzeichnet, daß** die Außenseite der Verpackungshülle eine textilähnliche Aufinachung erhält, und daß die Antihaft-Ausrüstung(124,152,162,172,175,176,182,185,192) auf der Innenseite der Verpackungshülle abschnittsweise vorgesehen wird.

9. Verfahren nach Anspruch 8, **dadurch gekennzeichnet, daß** die Verpackungshülle von einer Kunststoffolie(142) gebildet wird, deren Außenseite eine Prägung(145) erhält, die ihr ein textilähnliches Aussehen verleiht.

10. Verfahren nach Anspruch 9, **dadurch gekennzeichnet, daß** die Verpackungshülle von einer äußeren geprägten und einer inneren glatten Kunststoffolie gebildet wird.

11. Verfahren nach einem der vorhergehenden Ansprüche, **dadurch gekennzeichnet, daß** die Verpackungshülle(121) einen Vliesstoff(123) enthält.

12. Verfahren nach Anspruch 11, **dadurch gekennzeichnet, daß** der Vliesstoff(123) vorzugsweise an seiner Innenseite mit einer Kunststoffolie(122) verbunden wird.

13. Verfahren nach einem der vorangehenden Ansprüche, **dadurch gekennzeichnet, daß** wenigstens ein Randstreifen der Verpackungsmaterialbahn(91) von Antihaft-Material freigehalten wird.

14. Verfahren nach einem der vorangehenden Ansprüche, **dadurch gekennzeichnet, daß** die Gegenstände auf einen in der Mitte der Verpackungsmaterialbahn(91) angeordneten durchgehenden oder unterbrochenen Längsstreifen(22,32,42,45) mit Antihaft-Ausrüstung aufgelegt werden.

15. Verfahren nach einem der vorangehenden Ansprüche, **dadurch gekennzeichnet, daß** die Gegenstände auf ein Muster aus mehreren parallelen Längsstreifen(172,175,176) abwechselnd mit Antihaft-Ausrüstung und Längsstreifen(173,177,178), die frei von Antihaft-Ausrüstung sind, aufgelegt werden.

16. Verfahren nach einem der vorangehenden Ansprüche, **dadurch gekennzeichnet, daß** die Gegenstände auf einzelne Abschnitte mit Antihaft-Ausrüstung(2,33,93,103,124,152,162,182,185,192), die entsprechend den zu verpackenden Gegenständen bzw. deren Kleberbeschichtung angeordnet sind, aufgelegt werden.

17. Verfahren nach einem der vorangehenden Ansprüche, **dadurch gekennzeichnet, daß** zur Bildung der Antihaft-Ausrüstung Polysiloxane eingesetzt werden, die gehärtet werden.

18. Verfahren nach einem der vorangehenden Ansprüche, **dadurch gekennzeichnet, daß** als Verpackungsmaterialbahn(1,21,31,41,51) eine Kunststofffolie eingesetzt wird, die als Mono- oder Mehrschichtfolie ausgebildet ist und geprägt sein kann.

19. Verfahren nach einem der vorangehenden Ansprüche, **dadurch gekennzeichnet, daß** als Verpackungsmaterialbahn(1,21,31,41,51) ein vorzugsweise wenigstens einseitig mit Kunststoff beschichtetes Papier eingesetzt wird.

20. Verfahren nach einem der vorangehenden Ansprüche, **dadurch gekennzeichnet, daß** auf die von der Antihaft-Ausrüstung abgekehrte Seite der Verpackungsmaterialbahn ein Druckbild aufgebracht wird.

## Claims

1. Method of wrapping articles (4, 154, 164, 174, 184), which are equipped to be at least partially self-adhesive, by means of a wrapping material (91) provided in sections with an anti-adhesion finish (93), wherein the articles (4, 154, 164, 174, 184) are placed by their self-adhesive section on the sections with anti-adhesive finish of the wrapping material and the articles are subsequently covered by wrapping material, **characterised in that** for covering the articles (4) use is made of a further wrapping material web (95) and that provided at the edge of the wrapping is at least one free flap (52) which is separated from the wrapping material web (91) and is placed under one end section of the articles to be wrapped.

2. Method according to claim 1, **characterised in that** individual wrappers are separated after the articles (4, 154, 164, 174, 184) have been placed in or on.

3. Method according to claim 2, **characterised in that** the separation off of the individual wrappers is performed by a so-termed crimping (22, 23).

4. Method according to any one of the preceding claims, **characterised in that** perforation lines are provided for facilitating separation of individual wrappers.

5. Method according to any one of the preceding claims, **characterised in that** sticking plasters (4) provided with two adhesive sections are wrapped, wherein the sections with anti-adhesion finish (2, 103) are provided at least in the region of the adhesive sections.

6. Method according to any one of claims 1 to 4, **characterised in that** panty-liners (154, 164, 174, 184) are wrapped, which liners are provided with at least one self-adhesive section, in the region of which a section with anti-adhesion finish (124, 152, 162, 172, 175, 176, 182, 185, 192) is provided.

7. Method according to any one of the preceding claims **characterised in that** sanitary napkins (124, 152, 162, 172, 175, 176, 182, 185, 192) are wrapped, which napkins are provided with at least one self-adhesive section, in the region of which a section with anti-adhesion finish (124, 152, 162, 172, 175, 176, 182, 185, 192) is provided.

8. Method according to any one of the preceding claims, with an anti-adhesion coating (124, 152, 162, 172, 175, 176, 182, 185, 192) on the inner side of the wrapping envelope formed by the wrapping material, **characterised in that** the outer side of the wrapping element has a textile-like appearance and that the anti-adhesion finish (124, 152, 162, 172, 175, 176, 182, 185, 192) is provided on the inner side of the wrapping envelope in sections.

9. Method according to claim 8, **characterised in that** the wrapping envelope is formed by a plastics material film (142), the outer side of which has a stamping (145) imparting thereto a textile-like presentation.

10. Method according to claim 9, **characterised in that** the wrapping envelope is formed by an outer stamped plastics material film and an inner smooth plastics material film.

11. Method according to any one of the preceding claims, **characterised in that** the wrapping envelope 121) contains a non-woven material (123).

12. Method according to claim 11, **characterised in that** the non-woven material (123) is connected preferably at its inner side with a plastics material film (122).

13. Method according to any one of the preceding claims, **characterised in that** at least one edge strip of the wrapping material web (91) is kept free of anti-adhesion material.

14. Method according to any one of the preceding claims, **characterised in that** the articles are placed on a continuous or interrupted longitudinal strip (22, 32, 42, 45), which is arranged in the centre of the wrapping material web (91), with an anti-adhesion finish.

15. Method according to any one of the preceding claims, **characterised in that** the articles are placed on a pattern of several parallel longitudinal strips (172, 175, 176) with anti-adhesion finish and longitudinal strips (172, 177, 178) free of anti-adhesion finish, in alternation.

16. Method according to any one of the preceding claims, **characterised in that** the articles are placed on individual sections with anti-adhesion finish (2, 33, 93, 103, 124, 152, 162, 182, 185, 192), which are arranged in correspondence with the articles to be wrapped or the adhesive coating thereof.

17. Method according to any one of the preceding claims, **characterised in that** use is made of polysiloxanes, which are hardened, for forming the anti-adhesion finish.

18. Method according to any one of the preceding claims, **characterised in that** use is made of a plastics material film, which is formed as a single-layer or multilayer film and can be stamped, as wrapping material web (1, 21, 31, 41, 51).

19. Method according to any one of the preceding claims, **characterised in that** use is made of a paper, which is coated with plastics material preferably on at least one side, as wrapping material web (1, 21, 31, 41, 51).

20. Method according to any one of the preceding claims, **characterised in that** a printed image is applied to the side of the wrapping material web remote from the anti-adhesion finish.

## Revendications

1. Procédé de conditionnement d'objets (4, 154, 164, 174, 184) de réalisation au moins partiellement autocollante, au moyen d'un matériau d'emballage (91) muni, par zones, d'un dispositif anti-adhérence (93), lesdits objets (4, 154, 164, 174, 184) étant appliqués, par leur région autocollante, sur les régions à dispositif anti-adhérence dudit matériau d'emballage, et lesdits objets étant ensuite recouverts de matériau d'emballage, **caractérisé par le fait qu'**une nappe supplémentaire (95) de matériau d'emballage est utilisée pour recouvrir les objets (4) ; et **par le fait qu'**au moins une patte autonome (52), réservée sur le bord de l'emballage, est séparée d'avec la nappe (91) de matériau d'emballage, puis rabattue au-dessous de l'une des régions extrêmes desdits objets à conditionner.

2. Procédé selon la revendication 1, **caractérisé par le fait que** des emballages individuels sont dissociés après l'insertion ou la mise en applique respective des objets (4, 154, 164, 174, 184).

3. Procédé selon la revendication 2, **caractérisé par le fait que** la dissociation des emballages individuels est effectuée par le biais d'un « sertissage gaufré » (22, 23).

4. Procédé selon l'une des revendications précédentes, **caractérisé par le fait que** des lignes de perforations sont prévues pour faciliter la dissociation d'emballages individuels.

5. Procédé selon l'une des revendications précédentes, **caractérisé par** le conditionnement de pansements (4) présentant deux régions adhésives, les régions à dispositif anti-adhérence (2, 103) étant prévues au moins dans la zone desdites régions adhésives.

6. Procédé selon l'une des revendications 1 à 4, **caractérisé par** le conditionnement de couches protectrices intimes (154, 164, 174, 184) présentant au moins une région autocollante dans la zone de laquelle est prévue une région à dispositif anti-adhérence (124, 152, 162, 172, 175, 176, 182, 185, 192).

7. Procédé selon l'une des revendications 1 à 4, **caractérisé par** le
conditionnement de garnitures périodiques présentant au moins une région autocollante dans la zone de laquelle est prévue une région à dispositif anti-adhérence (124, 152, 162, 172, 175, 176, 182, 185, 192).

8. Procédé selon l'une des revendications précédentes, incluant un revêtement anti-adhérent (124, 152, 162, 172, 175, 176, 182, 185, 192) sur la face intérieure de l'enveloppe de conditionnement constituée du matériau d'emballage, **caractérisé par le fait que** la face extérieure de ladite enveloppe de conditionnement est pourvue d'un agencement offrant une similitude avec celui d'un textile ; et **par le fait que** le dispositif anti-adhérence (124, 152, 162, 172, 175, 176, 182, 185, 192) est prévu, par zones, sur la face intérieure de ladite enveloppe de conditionnement.

9. Procédé selon la revendication 8, **caractérisé par le fait que** l'enveloppe de conditionnement est constituée d'un feuil de matière plastique (142) dont la face extérieure présente une structure (145) à empreintes, qui lui confère une apparence analogue à celle d'un textile.

10. Procédé selon la revendication 9, **caractérisé par le fait que** l'enveloppe de conditionnement est composée d'un feuil extérieur de matière plastique muni d'empreintes, et d'un feuil intérieur de matière plastique lisse.

11. Procédé selon l'une des revendications précédentes, **caractérisé par le fait que** l'enveloppe de conditionnement (121) renferme un non-tissé (123).

12. Procédé selon la revendication 11, **caractérisé par le fait que** le non-tissé (123) est relié, de préférence par sa face intérieure, à un feuil de matière plastique (122).

13. Procédé selon l'une des revendications précédentes, **caractérisé par le fait qu'**au moins une bande marginale de la nappe (91) de matériau d'emballage est maintenue exempte de matériau anti-adhérent.

14. Procédé selon l'une des revendications précédentes, **caractérisé par le fait que** les objets sont mis en applique sur une bande longitudinale (22, 32, 42, 45) à dispositif anti-adhérence, continue ou discontinue, placée au centre de la nappe (91) de matériau d'emballage.

15. Procédé selon l'une des revendications précédentes, **caractérisé par le fait que** les objets sont mis en applique sur un patron composé, en alternance, de plusieurs bandes longitudinales parallèles (172, 175, 176) à dispositif anti-adhérence, et de bandes longitudinales (173, 177, 178) exemptes de dispositif anti-adhérence.

16. Procédé selon l'une des revendications précédentes, **caractérisé par le fait que** les objets sont mis en applique sur des régions individuelles à dispositif anti-adhérence (2, 33, 93, 103, 124, 152, 162, 182, 185, 192), qui sont respectivement agencées en concordance avec les objets à conditionner, ou avec le revêtement adhésif de ces derniers.

17. Procédé selon l'une des revendications précédentes, **caractérisé par le fait que** des polysiloxanes, soumis à durcissement, sont utilisés pour former le dispositif anti-adhérence.

18. Procédé selon l'une des revendications précédentes, **caractérisé par le fait qu'**un feuil de matière plastique, conçu comme un feuil monocouche ou multicouches et pouvant être muni d'empreintes, est utilisé en tant que nappe (1, 21, 31, 41, 51) de matériau d'emballage.

19. Procédé selon l'une des revendications précédentes, **caractérisé par le fait qu'**un papier revêtu de matière plastique, de préférence au moins sur une face, est utilisé en tant que nappe (1, 21, 31, 41, 51) de matériau d'emballage.

20. Procédé selon l'une des revendications précédentes, **caractérisé par le fait qu'**un motif imprimé est déposé sur la face de la nappe de matériau d'emballage qui est tournée à l'opposé du dispositif anti-adhérence.
